(19) Europäisches Patentamt
European Patent Office
Office européen des brevets

(11) Numéro de publication: **0 080 940 B1**

(12) **FASCICULE DE BREVET EUROPEEN**

(45) Date de publication du fascicule du brevet: **19.02.86**

(51) Int. Cl.⁴: **C 07 D 265/30**, A 61 K 31/535

(21) Numéro de dépôt: **82402147.1**

(22) Date de dépôt: **25.11.82**

(54) **Nouveaux dérivés de 2-phényl-morpholine et compositions thérapeutiques.**

(30) Priorité: **01.12.81 FR 8122507**

(43) Date de publication de la demande: **08.06.83 Bulletin 83/23**

(45) Mention de la délivrance du brevet: **19.02.86 Bulletin 86/8**

(84) Etats contractants désignés: **AT BE CH DE FR GB IT LI LU NL SE**

(56) Documents cités:
**FR - A - 1 535 615**
**FR - A - 2 471 378**
**GB - A - 851 311**
**US - A - 2 835 669**

**EUROPEAN JOURNAL OF MEDICINAL CHEMISTRY, CHIMICA THERAPEUTICA, vol. 11, no. 3, mai-juin 1976, pages 201-207; N. BUSCH et al.: "Tétrahydrooxazines-1,4. I. Nouvelle méthode de synthèse et étude de leur interaction avec les récepteurs tryptaminergiques D"**

**Le dossier contient des informations techniques présentées postérieurement au dépôt de la demande et ne figurant pas dans le présent fascicule.**

(73) Titulaire: **LABORATOIRE L. LAFON Société anonyme dite:, 1 rue Georges Médéric, F-94701 Maisons-Alfort (FR)**

(72) Inventeur: **Lafon, Louis, 5 rue de l'Alboni, F-75016 Paris (FR)**

(74) Mandataire: **Ranguis, Patrick Frédéric et al, Cabinet Beau de Loménie 55, rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

## Description

La présente invention concerne de nouveaux dérivés de 2-phényl-morpholine, à savoir la 2-phényl-4-éthyl-morpholine, la 2-o-tolyl-4-méthylmorpholine et leurs sels d'addition d'acide. Elle concerne également des compositions thérapeutiques.

On connaît un grand nombre de dérivés de 2-phénylmorpholine, notamment des brevets US-A-2 835 669 et US-A-2 997 469, de l'article de N. BUSCH et al., Eur. J. Med. Chem. Chimica Therapeutica 11 (n° 3) pages 201–207 (1976), des brevets français n° 7443M, FR-A-2 111 882) et FR-A-2 471 378). On sait également que plusieurs indications ont été envisagées ou préconisées pour les dérivés de 2-phényl-morpholine. Ces dérivés sont, notamment dans les publications précitées, présentés en tant qu'agents excitants ou stimulants du SNC, tranquillisants, anti-inflammatoires, analgésiques et hypotenseurs. Selon GB-A-83 1311 certains dérivés de 2-phényl-morpholine stimulent le SNC sans augmenter la pression sanguine, d'autres entraînent une diminution considerable de la pression sanguine. La 2-phényl-4- isopropyl-morpholine décrite dans FR-A-2 471 378 est un agent stimulant β-adrénergique. FR-A-1 535 615 divulgue la 2-phényl-4-méthyl-morpholine sans indication thérapeutique.

On vient de trouver de façon surprenante que les 2-phényl-4-éthyl-morpholine et 2-o-tolyl-4-méthyl-morpholine et leurs sels sont des agents antidépresseurs du SNC qui se distinguent avantageusement des composés de l'art antérieur. En particulier la 2-phényl-4-éthyl-morpholine et ses sels sont des agents antidépresseurs du SNC qui se distinguent des produits antérieurement connus notamment par le fait qu'ils présentent un effet stimulant α-adrénergique pré- et post-synaptique.

Les nouveaux composés de l'invention, qui appartiennent à la famille des dérivés de 2-phénylmorpholine répondant à la formule générale (I)

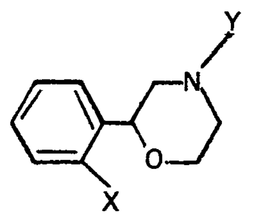

I

dans laquelle X et H et Y est $C_2H_5$, ou X=Y=$CH_3$.

En conséquence l'invention concerne particulièrement:

(i) la 2-phényl-4-éthyl-morpholine (X=H, Y=$C_2H_5$) et ses sels d'addition d'acide non toxiques; et

(ii) la 2-o-tolyl-4-méthyl-morpholine (X=Y=$CH_3$) et ses sels d'addition d'acide non toxiques.

Les composés préférés selon l'invention sont la 2-phényl-4-éthyl-morpholine et ses sels eu égard à l'effet stimulant α-adrénergique pré- et post-synaptique qu'ils présentent.

Parmi les acides minéraux et organiques qui conviennent pour salifier la base libre de formule I, on peut notamment citer les acides chlorhydrique, maléique, fumarique, aspartique, méthanesulfonique, et paratoluènesulfonique, le sel d'addition d'acide préféré étant, selon l'invention, le chlorhydrate.

La base libre de formule (I) peut être préparée selon une méthode connue en soi par application de mécanismes réactionnels classiques. Le procédé de préparation que l'on préconise ici comprend la cyclisation d'un dérivé de 1-phényl-2-[(N-hydroxyéthyl)- alkylamino]- 1-éthanol de formule (II) ci-après (où X et Y sont définis comme ci-dessus), en présence d'acide sulfurique, selon le schéma:

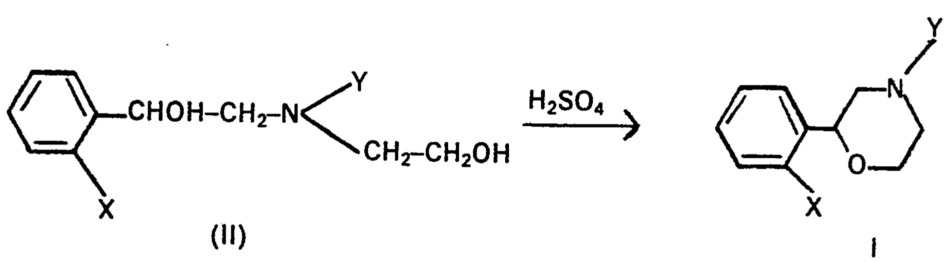


(II) I

Selon l'invention, on préconise une composition thérapeutique qui est caractérisée en ce qu'elle renferme, en association avec un excipient physiologiquement acceptable, au moins un composé choisi parmi l'ensemble constitué par la 2-phényl-4-éthyl-morpholine, la 2-o-tolyl-2-méthyl-morpholine, et leurs sels d'addition d'acide non toxiques, en tant que principe actif.

D'autres avantages et caractéristiques de l'invention seront mieux compris à la lecture qui va suivre d'exemples de préparation nullement limitatifs mais donnés à titre d'illustration.

Exemple 1

Chlorhydrate de 2-phényl-4-éthyl-morpholine (n° de code: CRL 40914).

On chauffe au reflux pendant 15 heures un mélange de 60 g (0,5 mole) d'oxyde de styrène, 222,5 g (2,5 moles) de 2-éthylamino-1-éthanol et 500 ml de méthanol. On évapore à sec, lave le résidu d'évaporation qui se présente sous la forme d'une huile avec 4× 200 ml d'hexane, puis sèche l'huile résultante sous vide au moyen d'un évaporateur rotatif. On coule l'huile ainsi séchée dans 125 ml de $H_2SO_4$ concentré refroidis par un bain

de glace. On laisse en contact pendant 1 heure, puis jette la milieu réactionnel dans un mélange contenant 500 ml d'eau + glace et 480 ml de NaOH 10N. On extrait à l'éther, lave la phase éthérée à l'eau, puis la sèche sur $MgSO_4$. On précipite le chlorhydrate attendu au moyen d'éthanol chlorhydrique. Par recristallisation dans le mélange acétone-éthanol (1:1) v/v, on obtient 40 g (rendement: 35% de CRL 40914. $F_{inst.}$ = 210–211 °C avec décomposition.

Analyse { % $Cl^-$ mesuré = 15,82%
% $Cl^-$ théorique = 15,60%.

Exemple 2

Chlorhydrate de 2-o-tolyl-4-méthyl-morpholine (n° de code CRL 40915), autre nomenclature: 2-(2-méthylphényl)-4-méthyl-morpholine.

On dissout 67 g (0,5 mole) de o-méthylacétophénone dans 100 ml d'acide acétique. On refroidit au moyen d'un bain de glace et coule 25,8 ml de brome. On laisse en contact pendant une heure et évapore à sec.

On reprend le résidu d'évaporation [qui renferme l'o-méthyl-ω-bromoacétophénone (o-$CH_3$–$C_6H_4$–$COCH_2Br$)] dans de l'acétone et coule le mélange résultant dans une solution de 187,5 g (2,5 moles) de 2-méthylamino-éthanol dans du méthanol. On chauffe le milieu réactionnel résultant au reflux pendant 4 heures. On évapore à sec, reprend le résidu d'évaporation à l'eau et extrait avec de l'acétate d'éthyle. On lave à l'eau, extrait la phase acétate d'éthyle par un mélange de 500 ml d'eau et 60 ml d'HCl concentré ($d_4^{15\,°C}$ = 1,19 g/$cm^3$). On lave la phase aqueuse avec de l'acétate d'éthyle et la neutralise avec NaOH jusqu'à pH 11, extrait à l'acétate d'éthyle, lave à l'eau, sèche sur $MgSO_4$, filtre et évapore à sec.

On reprend le résidu d'évaporation [qui renferme la N-β-hydroxyéthyl-N- méthyl-o-méthylphénacyl-amine] par 500 ml de méthanol, ajoute 38 g (1 mole) de $NaBH_4$. On laisse en contact une nuit. On détruit par 60 ml d'acide acétique, évapore à sec, reprend à l'eau, ajuste à pH 11 avec NaOH, extrait à l'acétate d'éthyle lave la phase organique à l'eau, sèche sur $MgSO_4$, filtre, puis évapore à sec.

On coule le résidu d'évaporation ainsi obtenu [qui renferme le 1-o-tolyl-2- (N-β-hydroxyéthyl)-N-méthyl)- amino-1-éthanol] dans 125 ml d'acide sulfurique concentré refroidis au bain de glace. On laisse en contact pendant 1 heure, puis jette le milieu réactionnel dans un mélange de 460 ml de lessive de soude, 500 ml d'eau et de glace. On extrait à l'éther, lave à l'eau, sèche la phase éthérée avec $MgSO_4$, filtre puis précipite le chlorhydrate attendu au moyen d'éthanol chlorhydrique. Par recristallisation dans le mélange éther-éthanol (1:1) v/v on obtient 10 g (rendement: 9%) de CRL 40915. $F_{inst.}$ = 192 °C (avec décomposition).

Analyse { %$Cl^-$ mesuré = 15,97%
% $Cl^-$ théorique = 15,60%

On a résumé ci-après les résultats des essais qui ont été entrepris chez l'animal avec le CRL 40914, ce produit étant administré, sauf indications contraires, en solution dans l'eau (à pH 5) par voie intrapéritonéale sous un volume de 20 ml/kg chez la souris mâle et de 5 ml/kg chez le rat mâle.

I – Toxicité

Chez la souris mâle, la DL-O (dose maximale non mortelle) par voie i.p. est supérieure à 128 mg/kg.

II – Comportement global et réactivité

Des lots de 3 animaux sont observés avant, puis 15 min, 30 min, 1 h, 2 h, 3 h et 24 h après l'administration de CRL 40914. On observe ce qui suit:

1 – Souris

à la dose de 64 mg/kg:

– excitation pendant 1 à 2 h avec manifestation de mouvements stéréotypés,

– piloérection, salivation et lacrymation pendant 1 h,

– mydriase pendant 1 h;

– polypnée pendant 2 h;

à la dose de 16 mg/kg:

– excitation pendant 30 min suivie d'une hyporéactivité au toucher,

– lacrymation pendant 1 h,

– hypothermie (−2,8 °C) pendant 2 à 3 h,

– mydriase pendant 1 h;

à la dose de 4 mg/kg:

– sédation pendant 30 à 60 min,

– hypothermie (−2,2 °C) pendant 1 h,

– mydriase pendant 1 heure;

à la dose de 2 mg/kg:

– sédation pendant 30 à 60 min,

– hypothermie (−3,6 °C) pendant 1 à 2 h,

– mydriase pendant 1 h.

2 – Rat

à la dose de 32 mg/kg:

– sédation pendant 3 h avec diminution de la réactivité au toucher et du tonus musculaire,

– augmentation de la réaction de peur pendant 3 h,

– piloérection (pendant 3 h), salivation (pendant 1 h) et lacrymation (pendant 1 h),

– mydriase pendant plus de 3 h;

à la dose de 8 mg/kg:

– sédation pendant 2 h avec diminution de la réactivité au toucher mais augmentation de la réaction de peur,

– piloérection pendant 1 à 2 h,

– mydriase pendant 2 h;

à la dose de 2 mg/kg:

– sédation pendant 1 h,

– mydriase pendant 1 à 2 h;

à la dose de 0,5 mg/kg:

– mydriase pendant 1 à 2 h.

III – Action sur la température

Des lots de 6 souris reçoivent le CRL 40914; la température rectale est notée toutes les 30 min. On observe qu'aux doses de 0,25 mg/kg, 1 mg/kg, 4 mg/kg et 16 mg/kg le CRL 40914 entraîne une hypothermie maximale après 30 min, cette hypothermie durant pendant 90 à 120 min.

A dose plus élevée (64 mg/kg) c'est au contraire une hyperthermie qui apparaît à 30 min et qui fait ensuite place à une hypothermie modérée.

IV – Recherche de mouvements stéréotypes

Des lots de 6 rats reçoivent une injection intrapéritonéale de CRL 40914 ou d'amphétamine puis sont immédiatement placés dans des boîtes de petites dimensions où leur comportement stéréotypé est coté de 0 à 3 toutes les 10 min jusqu'à extinction de l'effet.

On observe que le CRL 40914 ne provoque pas de stéréotypies à la dose de 32 mg/kg. A la forte dose de 64 mg/kg, il entraîne l'apparition de mouvements stéréotypés d'intensité et de durée comparables à ceux observés après administration de 1 mg/kg d'amphétamine. A la très forte dose de 128 mg/kg, cet effet est encore plus net.

V – Interaction avec l'apomorphine

1 – Souris

Des lots de 6 souris reçoivent le CRL 40914 une demi-heure avant l'injection sous-cutanée d'apomorphine, à la dose de 1 mg/kg ou 16 mg/kg.

On observe qu'à forte dose (16 mg/kg, 64 mg/kg) le CRL 40914 s'oppose à l'action hypothermisante de l'apomorphine et semble augmenter les indices de verticalisation et de stéréotypies. On retrouve donc l'effet du CRL 40914 auxdites doses sur la température.

2 – Rat

Le CRL 40914 est administré aux rats une demi-heure avant l'injection sous-cutanée de 0,5 mg/kg d'apomorphine.

Aux doses de 0,5 mg/kg, 2 mg/kg et 8 mg/kg le CRL 40914 semble diminuer modérément l'intensité des téréotypies induites par l'apomorphine.

A dose plus élevée (32 mg/kg) il semble au contraire provoquer une potentialisation discrète des stéréotypies.

VI – Interaction avec l'amphétamine

L'amphétamine (2 mg/kg) est injectée par voie intrapéritonéale à des lots de 12 rats, une demi-heure après l'administration de CRL 40914.

On observe qu'aux doses de 8 mg/kg et 32 mg/kg le CRL 40914 diminue nettement l'intensité des téréotypies amphétaminiques.

VII – Interaction avec la réserpine

Quatre heures après l'injection intrapéritonéale de 2,5 mg/kg de réserpine, des lots de 6 souris reçoivent le CRL 40914.

On note qu'aux doses de 4 mg/kg mais surtout 16 mg/kg et 64 mg/kg, le CRL 40914 s'oppose à l'hypothermie et au ptosis réserpinique.

VIII – Interaction avec l'oxotrémorine

Le CRL 40914 est administré à des lots de 6 souris une demi-heure avant l'injection intrapéritonéale de 0,5 mg/kg d'oxotrémorine.

1 – Action sur la température.

Aux doses de 16 mg/kg et 64 mg/kg, le CRL 40914 combat l'effet hypothermisant de l'oxotrémorine. Il exerce sur la température un effet intrinsèque biphasique en fonction des doses.

2 – Action sur les tremblements.

A la forte dose (64 mg/kg) le CRL 40914 diminue l'intensité des tremblements dus à l'oxotrémorine.

3 – Action sur les symptômes cholinergiques périphériques.

Le CRL 40914 laisse inchangés les signes de stimulation cholinergique périphériques provoqués par l'oxotrémorine.

IX – Action sur le test des quatre plaques, la traction et l'électrochoc.

Le test est pratiqué sur des lots de 10 souris, une demi-heure après l'administration de CRL 40914.

Le CRL 40914 entraîne à faible dose une diminution, et à forte dose une augmentation du nombre de passages punis, il ne provoque pas de déficit moteur majeur, et à forte dose (64 mg/kg) il aggrave les effets létaux de l'électrochoc.

X – Action sur la motilité spontanée

Une demi-heure après avoir reçu le CRL 40914, des souris sont placées en actimètre où leur motilité est enregistrée pendant 30 min.

On constate que le CRL 40914 entraîne dès la dose de 0,125 mg/kg une diminution de l'activité motrice spontanée de la souris. Cet effet atteint un maximum pour des doses comprises entre 0,5 mg/kg et 16 mg/kg. Au-delà l'effet dépresseur moteur diminue puis fait place (64 mg/kg) à une hyperactivité nette.

XI – Action vis-à-vis de quelques comportements perturbés par divers agents.

1 – Motilité réduite par habituation à l'enceinte.

Après 18 h de séjour dans les actimètres, les souris (6 par dose, 12 témoins) reçoivent le CRL 40914. Elles sont aussitôt replacées dans leurs enceintes respectives et, une demi-heure plus tard, on enregistre leur motilité pendant 30 min.

On observe qu'à forte dose (64 mg/kg) le CRL 40914 entraîne une reprise très nette de l'activité motrice chez la souris habituée à son enceinte.

2 – Motilité réduite par agression hypoxique.

Une demi-heure après avoir reçu le CRL 40914, les souris (10 par dose, 20 témoins) sont soumises à une anoxie hypobare aiguë [dépression de 600 mm Hg (soit environ $8 \times 10^4$ pascals) en 90 s puis détente de 45 s] puis elles sont placées en actimètre où leur motilité est enregistrée pendant 10 min.

Chez la souris dont la motilité a été déprimée à la suite d'un séjour bref dans une enceinte à pression réduite, le CRL 40914 entraîne des modifications de l'activité motrice qui reflètent son action sur la motilité spontanée.

3 – Anoxie asphyxique.

Des lots de 10 souris reçoivent le CRL 40914 une demi-heure avant l'administration intrapéritonéale de 32 mg/kg de triiodoéthylate de gallamine (agent curarisant de référence).

On constate qu'aux doses de 1 mg/kg, 4 mg/kg et 16 mg/kg, le CRL 40914 entraîne un retard d'apparition des convulsions et, à un degré moindre, de la mort consécutive à une anoxie asphyxique provoquée par un curarisant. A dose plus élevée, cet effet disparaît.

XII – Interaction avec le barbital

Des lots de 10 souris reçoivent le CRL 40914 une demi-heure avant l'injection intrapéritonéale de 220 mg/kg de barbital sodique.

Aux doses de 4 mg/kg et 16 mg/kg, le CRL 40914 semble prolonger modérément la durée du sommeil induit par le barbital, alors qu'à dose plus élevée (64 mg/kg) il exerce un antagonisme net.

XIII – Action sur le «Désespoir comportemental»

Une demi-heure après avoir reçu le CRL 40914 ou la désipramine, des lots de 10 souris sont plongés dans un bécher rempli d'eau sur une hauteur de 6 cm. On note la durée d'immobilité de la souris entre la 2ème et la 6ème min suivant son immersion.

Dès la dose de 4 mg/kg, le CRL 40914 diminue la durée d'immobilité de la souris placée en immersion; l'effet est particulièrement net à 64 mg/kg.

XIV – Interaction avec la yohimbine

1 – Température

Des lots de 12 souris reçoivent une injection intrapéritonéale de 0,5 mg/kg de yohimbine, une demi-heure avant l'administration de CRL 40914.

On observe qu'à la dose de 0,5 mg/kg, la yohimbine ne modifie pas la température rectale de la souris mais qu'elle empêche l'apparition de l'hypothermie induite par 1 mg/kg et 16 mg/kg de CRL 40914.

2 – Motilité spontanée

Des lots de 12 souris reçoivent la yohimbine (0,5 mg/kg i.p.) et le CRL 40914 respectivement 60 et 30 min avant d'être placées en actimètre où leur motilité est enregistrée pendant une demi-heure.

On constate que le yohimbine ne modifie pas l'activité motrice spontanée; elle s'oppose à l'hypomotilité produite par le CRL 40914 à la dose de 1 mg/kg mais ne modifie pas celle provoquée par 16 mg/kg de CRL 40914.

XV – Action sur l'agressivité intergroupe

On constate que le CRL 40914 a un effet antiagressif chez la souris mâle lors de l'étude de l'agressivité intergroupe, en ce sens qu'il supprime les combats entre les souris de 2 groupes différents à la dose de 4 mg/kg, et diminue la durée de contact entre les souris à la dose de 64 mg/kg. Cet effet pourrait résulter de la sédation qui se manifeste à faible dose (4 mg/kg) et de l'excitation motrice qui se manifeste à forte dose (64 mg/kg).

XVI – Action sur le systeme cardiovasculaire

Six rats génétiquement hypertendus à cathéter artériel fémoral implanté reçoivent le CRL 40914 à la dose de 20 mg/kg par voie buccale.

On observe que le CRL 40914 est modérément hypotenseur. Il diminue progressivement la pression artérielle; l'effet maximal est obtenu 7 h après l'ingestion du produit; la pression artérielle passe de 165 à 135 mm Hg (c'est-à-dire de $2,2 \times 10^4$ à $1,8 \times 10^4$ pascals) soit une variation de – 18%. La fréquence cardiaque diminue: elle passe de 350 battements/minuts à un minimum de 270 battements/minute après 1,5 h, elle remonte progressivement puis redescend; après 24 h, pression artérielle et fréquence cardiaque sont revenues à leur valeur témoin.

Il résulte de l'ensemble des résultats des essais relatifs au SNC donnés aux points II–XII et XIV–XV ci-dessus, que le CRL 40914 (i) présente un effet biphasique en fonction des doses utilisées:

a) sédation, hypomotilité et hypothermie aux faibles doses; et

b) excitation, hypermotilité, hyperthermie, stéréotypies et antagonisme du sommeil barbiturique aux doses élevées; et (ii) agit en tant que stimulant α-adrénergique présynaptique aux faibles doses et postsynaptique aux doses élevées.

On a résumé ci-après les résultats des essais qui ont été entrepris chez l'animal avec le CRL 40915, les modalités opératoires étant celles données cidessus pour le CRL 40914.

I – Toxicité

Chez la souris mâle, la DL-O (dose maximale non mortelle) par voie i.p. est supérieure à 256 mg/kg, et la DL-50 par voie i.p. est de l'ordre de 500 mg/kg environ.

II – Comportement global et réactivité

1 – Souris

à la dose de 128 mg/kg:

– convulsions pendant 15 minutes

– sédation avec diminution de la réaction d'agressivité pendant 2 heures,

– hyporéactivité au toucher pendant 15 minutes,

– salivation et lacrymation pendant 30 minutes, et

– hypothermie (–2,9 °C) pendant 2 heures;

à la dose de 32 mg/kg:

– sédation pendant 1 heure,

– hypothermie (–2,8 °C) pendant 1 heure;

à la dose de 8 mg/kg:

– sédation fugace pendant 15 à 30 minutes,

– hypothermie (–1,5 °C) pendant 1 heure;

à la dose de 2 mg/kg:

– pas de symptômes nets.

2 – Rats

à la dose de 64 mg/kg:

– sédation pendant 1 à 2 heures,

– piloérection pendant 1 heure,

– hypothermie (–1,9 °C) pendant 30 à 60 minutes,

– mydriase pendant 1 heure;

à la dose de 16 mg/kg:

– sédation pendant 1 à 2 heures,

– piloérection

– mydriase pendant 30 à 60 minutes;

à la dose de 4 mg/kg:

– sédation pendant 30 minutes,

– mydriase modérée pendant 30 minutes;

à la dose de 1 mg/kg:

– pas de signes particuliers.

III – Interaction avec l'apomorphine

1 – Souris

Le CRL 40915 exerce pour des doses voisines de 32 mg/kg un effet hypothermisant intrinsèque. Il s'oppose aux doses de 32 mg/kg et 128 mg/kg à l'action hypothermisante de l'apomorphine, et à 128 mg/kg il diminue les indices de verticalisation et de stéréotypies induites par la faible dose (1 mg/kg) d'apomorphine.

2 – Rat

Le CRL 40915 ne modifie pas les stéréotypies produites par l'apomorphine chez le rat.

IV – Interaction avec l'amphétamine

Les stéréotypies amphétaminiques ne sont pas modifiées par le CRL 40915.

V – Interaction avec la réserpine

A la forte dose (128 mg/kg) la CRL 40915 entraîne une aggrevation de l-hypothermie réserpinique; le ptosis déjà maximal n'est pas modifié.

VI – Interaction avec l'oxotrémorine

1 – Action sur la température

Le CRL 40915 aux doses de 32 mg/kg et 128 mg/kg exerce un effet hypothermisant intrinsèque, mais s'oppose à l'hypothermie induite par l'oxotrémorine.

2 – Action sur les tremblements

Les tremblements induits par l'oxotrémorine semblent partiellement antagonisés par la forte dose (128 mg/kg) de CRL 40915.

3 – Action sur les symptômes cholinergiques périphériques

Le CRL 40915 laisse inchangés les signes de stimulation cholinergique périphérique dus à l'oxotrémorine.

VII – Action sur le test des quatre plaques, la traction et l'électrochoc

Aux doses de 32 mg/kg et 128 mg/kg, le CRL 40915 diminue le nombre de passages punis, il ne provoque pas de déficit moteur majeur mais s'oppose aux effets convulsivants de l'électrochoc.

VIII – Action sur la motilité spontanée

Aux doses de 32 mg/kg et 128 mg/kg, le CRL 40915 entraîne une diminution importante de l'activité motrice spontanée de la souris.

IX – Action vis-à-vis de quelques comportements perturbés par divers agents

1 – Motilité réduite par habituation à l'enceinte

Le CRL 40915 ne provoque pas de reprise de l'activité chez la souris habituée à son enceinte.

2 – Motilité réduite par agression hypoxique

Le CRL 40915 n'entraîne pas d'amélioration de la récupération motrice chez les souris dont la motilité a été déprimée à la suite d'un séjour bref dans une enceinte à pression réduite. Aux doses de 32 mg/kg et 128 mg/kg, on note au contraire une aggravation probablement en relation avec l'effet du CRL 40915 sur la motilité spontanée.

3 – Anoxie asphyxique

Aux doses de 8 mg/kg 32 mg/kg et 128 mg/kg, le CRL 40915 retarde l'apparition des convulsions et de la mort consécutives à une anoxie asphyxique provoquée par un curarisant (triiodoéthylate de gallamine).

Il résulte de ces essais que le profil neuropsychopharmacologique du CRL 40915 est caractérisé par une sédation importante n'apparaissant pas progressivement avec l'augmentation des doses mais se produisant en tout ou rien: pas d'effet sédatif à une dose donnée, sédation très importante à la dose supérieure utilisée.

L'hypothermie (qui semble moins importante à dose élevée qu'à dose moyenne), l'hypomotilité, la diminution du nombre de passages punis au test des quatre plaques, l'aggravation des effets akinésiants de l'hypoxie, le retard d'apparition des convulsions et de la mort, après anoxie asphyxique, sont autant d'effets que l'on peut rattacher à la sédation.

Par ailleurs à dose élevée, le CRL 40915 s'oppose nettement aux hypothermies induites par l'apomorphine et l'oxotrémorine mais aggrave l'effet hypothermisant de la réserpine.

Enfin, à dose élevée, le CRL 40915 entraîne l'apparition de salivation et de piloérection (qui pourraient être en relation avec une stimulation α-adrénergique post-synaptique périphérique) et de convulsions qui ne se manifestent que pendant les 15 à 30 minutes qui suivent l'administration du produit.

En clinique, le CRL 40914 administré à l'homme par voie orale [2 à 3 comprimés ou gélules (renfermant chacun 10 à 20 mg de principe actif) par jour], a donné d'excellents résultats notamment en tant qu'agent antidépresseur du SNC.

**Revendications pour les Etats contractants: BE, CH, DE, FR, GB, IT, LI, LU, NL, SE**

1. Dérivé de 2-phényl-morpholine, utile notamment en thérapeutique, caractérisé en ce qu'il est choisi parmi l'ensemble constitué par:

(i) la 2-phényl-4-éthyl-morpholine et ses sels d'addition d'acide, et

(ii) la 2-o-tolyl-4-méthyl-morpholine et ses sels d'addition d'acide.

2. Composé selon la revendication 1, caractérisé en ce qu'il est choisi parmi l'ensemble constitué par la 2-phényl-4-éthyl-morpholine et ses sels d'addition d'acide.

3. Composé selon la revendication 1, caractérisé en ce qu'il est choisi parmi l'ensemble constitué par la 2-o-tolyl-4-méthyl-morpholine et ses sels d'addition d'acide.

4. Composition thérapeutique, caractérisée en ce qu'elle renferme, en association avec un excipient physiologiquement acceptable, une quantité pharmaceutiquement efficace d'au moins un composé choisi parmi l'ensemble constitué par la 2-phényl-4-éthyl-morpholine, la 2-o-tolyl-4-méthyl-morpholine et leurs sels d'addition d'acide non toxiques.

**Revendications pour l'Etat contractant: AT**

1. Utilisation d'un dérivé de 2-phényl-morpholine choisi parmi l'ensemble constitué par:

(i) la 2-phényl-4-éthyl-morpholine et ses sels d'addition d'acide, et

(ii) la 2-o-tolyl-4-méthyl-morpholine et ses sels d'addition d'acide pour la fabrication d'un médicament.

2. Utilisation selon la revendication 1, caractérisée en ce qu'il est choisi parmi l'ensemble constitué par la 2-phényl-4-éthyl-morpholine et ses sels d'addition d'acide.

3. Utilisation selon la revendication 1, caractérisée en ce qu'il est choisi parmi l'ensemble constitué par la 2-o-tolyl-4-méthyl-morpholine et ses sels d'addition d'acide.

4. Utilisation selon l'une des revendications 1 à 3, en association avec un excipient physiologiquement acceptable, d'une quantité pharmaceutiquement efficace d'au moins un composé choisi parmi l'ensemble constitué par la 2-phényl-4-éthyl-morpholine, la 2-o-tolyl-4-méthyl-morpholine et leurs sels d'addition d'acide non toxiques.

**Patentansprüche für die Vertragsstaaten: BE, CH, DE, FR, GB, IT, LI, LU, NL, SE**

1. Insbesondere in der Therapie nützliches 2-Phenyl-morpholin-Derivat, dadurch gekennzeichnet, dass es ausgewählt ist aus der Gruppe bestehend aus:

(i) 2-Phenyl-4-äthyl-morpholin und seinen Säureadditionssalzen, und

(ii) 2-o-Tolyl-4-methyl-morpholin und seinen Säureadditionssalzen.

2. Verbindung nach Anspruch 1, dadurch gekennzeichnet, dass sie ausgewählt ist aus der Gruppe bestehend aus 2-Phenyl-4-äthyl-morpholin und seinen Säureadditionssalzen.

3. Verbindung nach Anspruch 1 dadurch gekennzeichnet, dass sie ausgewählt ist aus der Gruppe bestehend aus 2-o-Tolyl-4-methyl-morpholin und seinen Säureadditionssalzen.

4. Therapeutische Zusammensetzung, dadurch gekennzeichnet, dass sie in Verbindung mit einem physiologisch akzeptablen Exzipienten eine pharmazeutisch wirksame Menge von mindestens einer Verbindung, ausgewählt aus der Gruppe bestehend aus 2-Phenyl-4-äthyl-morpholin, 2-o-Tolyl-4-methyl-morpholin und deren nichttoxischen Säureadditionssalzen, enthält.

**Patentansprüche für den Vertragsstaat: AT**

1. Verwendung eines 2-Phenyl-morpholin-Derivats, ausgewählt aus der Gruppe bestehend aus:

(i) 2-Phenyl-4-äthyl-morpholin und seinen Säureadditionssalzen, und

(ii) 2-o-Tolyl-4-methyl-morpholin und seinen Säureadditionssalzen, zur Herstellung eines Medikaments.

2. Verwendung nach Anspruch 1 dadurch gekennzeichnet, dass es ausgewählt ist aus der Gruppe bestehend aus 2-Phenyl-4-äthyl-morpholin und seinen Säureadditionssalzen.

3. Verwendung nach Anspruch 1, dadurch gekennzeichnet, dass es ausgewählt ist aus der Gruppe bestehend aus 2-o-Tolyl-4-methyl-morpholin und seinen Säureadditionssalzen.

4. Verwendung nach einem der Ansprüche 1 bis 3, einer pharmazeutisch wirksamen Menge von mindestens einer Verbindung, ausgewählt aus der Gruppe bestehend aus 2-Phenyl-4-äthylmorpholin, 2-o-Tolyl-4-methyl-morpholin und deren nichttoxischen Säureadditionssalzen, in Verbindung mit einem physiologisch akzeptablen Exzipienten.

**Claims for the Contracting States: BE, CH, DE, FR, GB, IT, LI, LU, NL, SE**

1. 2-phenyl-morpholine derivative, useful in particular in therapeutics, characterized in that it is selected from the group constituted by:

(i) the 2-phenyl-4-ethyl-morpholine and its acid addition salts, and

(ii) the 2-o-tolyl-4-methyl-morpholine and its acid addition salts.

2. Compound according to claim 1, characterized in that it is selected from the group constituted by the 2-phenyl-4-ethyl-morpholine and its acid addition salts.

3. Compound according to claim 1, characterized in that it is selected from the group constituted by the 2-o-tolyl-4-methyl-morpholine and its acid addition salts.

4. Therapeutical composition, characterized in that it contains, in combination with a physiologically acceptable excipient, a pharmaceutically efficient quantity of at least one compound from the group constituted by the 2-phenyl-4-ethyl-morpholine, the 2-o-tolyl-4-methyl-morpholine and their non-toxic acid addition salts.

**Claims for the Contracting State: AT**

1. Use of a 2-phenyl-morpholine derivative selected from the group constituted by:

(i) 2-phenyl-4-ethyl-morpholine and its acid addition salts, and

(ii) 2-o-tolyl-4-methyl-morpholine and its acid addition salts, for the preparation of a drug.

2. Use according to claim 1, characterized in that it is selected from the group constituted by the 2-phenyl-4-ethyl-morpholine and its acid addition salts.

3. Use according to claim 1, characterized in that it is selected from the group constituted by the 2-o-tolyl-4-methyl-morpholine and its acid addition salts.

4. Use according to one of claims 1 to 3, in combination with a physiologically acceptable excipient, of a pharmaceutically efficient quantity of at least one compound selected from the group constituted by the 2-phenyl-4-ethyl-morpholine, the 2-o-tolyl-4-methyl-morpholine and their non-toxic acid addition salts.